(19) Europäisches Patentamt
European Patent Office
Office européen des brevets

(11) **EP 2 261 668 A1**

(12) **EUROPEAN PATENT APPLICATION**

(43) Date of publication:
**15.12.2010 Bulletin 2010/50**

(51) Int Cl.:
***G01N 33/66*** *(2006.01)*

(21) Application number: **09162517.8**

(22) Date of filing: **11.06.2009**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK TR**<br>Designated Extension States:<br>**AL BA RS**<br><br>(71) Applicant: **Nederlandse Organisatie voor Toegepast -Natuurwetenschappelijk Onderzoek TNO 2628 VK Delft (NL)** | (72) Inventors:<br>• **Havenaar, Robert**<br>  **3705 BC Zeist (NL)**<br>• **Minekus, Mans**<br>  **3985 LB Werkhoven (NL)**<br>• **Bellmann, Susann Christine**<br>  **6708 LT Wageningen (NL)**<br><br>(74) Representative: **Hatzmann, Martin Vereenigde Johan de Wittlaan 7 2517 JR Den Haag (NL)** |

(54) **Method for predicting glycaemic response and use thereof**

(57)    The present invention relates to an *in vitro* method for predicting the *in vivo* glycaemic response to carbohydrates in a food. Provided is a method wherein a food is digested *in vitro* in more than one compartment.

Also provided is the use of such a method for determining the suitability of a food for a subject who would benefit from a normalization of blood glucose levels.

**Description**

INTRODUCTION

The present invention relates to an *in vitro* method for predicting the *in vivo* glycaemic response to carbohydrates in a food. Provided is a method wherein a food is digested *in vitro* in more than one compartment. Also provided is the use of such a method for determining the suitability of a food for a subject who would benefit from a normalization of blood glucose levels.

BACKGROUND OF THE INVENTION

The effects of carbohydrates in a food on *in vivo* blood glucose levels can be measured by determining the food's glycaemic response or glycaemic index (also glycemic index), abbreviated as GI (Jenkins et al., American Journal of Clinical Nutrition 1981 vol. 34: 362-366). First, an amount of the food to be tested is determined so that the amount comprises a fixed portion of carbohydrates, usually 50 grams. Following the *in vivo* ingestion and digestion of this pre-determined amount of test food over time, a two hour blood glucose response curve can be determined. The area under the curve (AUC) is then divided by the AUC of a reference food and multiplied by 100. The outcome of this calculation denotes the GI of the food. The current validated methods use glucose as the reference food, giving it a GI value of 100 by definition. The average GI value for a test food is calculated from data collected in 10 human subjects (Brouns et al., Nutrition Research Reviews 2005 vol. 18: 145-171).

Carbohydrates with a high GI (70 and above) are carbohydrates that break down rapidly during digestion, causing glucose to be released into the bloodstream quickly and thus eliciting a large glycaemic response. Examples of foods containing such carbohydrates are cornflakes, baked potato and white bread. Carbohydrates with a low GI (55 or less) are less digestible carbohydrates or carbohydrates that break down slowly, resulting in a lower or gradual release of glucose into the bloodstream. Examples of foods containing such carbohydrates are grainy breads such as pumpernickel, most fruits and vegetables and brown rice. Whole wheat products, table sugar, beer and most species of white rice are examples of foods containing carbohydrates with a medium GI (56-69).

Low glycaemic diets have been reported to improve both glucose and lipid levels in people with diabetes mellitus (type 1 and 2), to result in benefits in weight control because they help control appetite and hunger and to reduce insulin levels and insulin resistance.

Furthermore, several lines of scientific evidence have shown that individuals who followed a low GI diet over many years were at a significantly lower risk than others for developing both type 2 diabetes mellitus and coronary heart disease. High blood glucose levels or repeated glycaemic "spikes" following a meal may promote these diseases by increasing oxidative damage to the vasculature, and also by the direct increase in insulin levels (Temelkova-Kurktschiev et al., Diabetes Care 2000 vol. 23(12): 1830-1834). In the past, postprandial hyperglycemia has been considered a risk factor mainly associated with diabetes. However, more recent research shows that it also presents an increased risk for atherosclerosis in the non-diabetic population (Balkau et al., Diabetes Care 1998 vol. 21(3): 360-367).

Thus, foods with a low GI (i.e., foods that elicit a small glycaemic response) are considered to provide significant health benefits in general, especially for subjects suffering from or having increased risk for developing syndromes such as glucose intolerance, insulin resistance, diabetes mellitus, syndrome X or metabolic syndrome, cardiovascular disease and obesity. It is therefore often recommended that consumers modify their overall diets such that the relative amount of low GI foods is increased, at the expense of high GI foods.

Foods with a high GI (i.e., food that elicits a large glycaemic response) may however also provide benefits, depending on their intended use. For example, as such foods cause a more rapid rise in blood glucose levels they are suitable for a person in need of energy recovery after endurance exercise or for a person suffering from diabetes mellitus who is experiencing hypoglycemia.

As glycaemic response values can be used to assist consumers and instances such as health care providers in selecting foods and possibly reducing the risk of certain diseases, methods to accurately predict the *in vivo* glycaemic response values or GI of carbohydrates in a food are needed.

The "gold standard" for calculating a food's *in vivo* GI is to measure the changes in blood glucose levels that occur over time after consumption and digestion of a fixed amount of that food. The obtained *in vivo* glucose response is then compared to the *in vivo* glucose response of a reference food with a known GI, usually glucose. As mentioned above, the average GI value for a test food is typically calculated from data collected in 10 human subjects.

However, this *in vivo* method suffers from considerable drawbacks. Not only does it require participation of human subjects, it also requires overnight fasting and frequent invasive blood sampling. Also, the method comprises not one but two tests as the glucose response to the reference food has to be measured as well. Thus, it may be considered unethical.

The method is also rather time consuming and costly. Most importantly, compliance of the participants to the test protocol is difficult to guarantee, casting doubts about the reliability of the obtained results. Alternative methods are therefore needed that avoid participation of human subjects and require less time and money, without loss of accuracy in calculating glycaemic responses as compared to *in vivo* conditions.

Although there has been considerable interest in developing *in vitro* protocols only a very few have actually been established, of which the best known is the method by Englyst *et al.* The initial method of Englyst *et al.* involved the measurement of glucose, released from a crushed test food during incubation at 37 °C with HCl and a mixture of digestive enzymes, using a calorimetric assay to determine the final glucose level (Englyst et al., British Journal of Nutrition 1996 75: 327-337). It was modified a few years later by replacing the calorimetric assay with an HPLC assay to improve reproducibility (Englyst et al., American Journal of Clinical Nutrition 1999 69: 448-454), and it has remained unchanged ever since. The final *in vitro* method by Englyst *et al.* using the HPLC endpoint is hereinafter referred to as the "Englyst method".

The Englyst method also suffers from drawbacks. The conditions under which the tested food is digested are not comparable to *in vivo* digesting conditions. For example, the first digesting step involves crushing or mincing a food sample to an "as eaten" state without any enzymatic pre-digestion of the food, such as by amylase. Also, after addition of HCl and pepsin the minced food mix is only buffered back to pH 5.2 instead of to at least neutral value (pH = 7-8) as would occur in an *in vivo* intestinal tract. Enzymes for digesting the carbohydrates in the food sample are not added until after the pH has been buffered.

Furthermore, the Englyst method does not involve different digestive compartments. All the reacting substances are mixed together in one and the same tube. Thus, the typical conditions that are met by the food in the various digestive compartments *in vivo* are not provided. Moreover, the Englyst method does not account for peristaltic movement of the food.

Also, only two samples are taken during the whole incubation procedure. The first sample, $G_{20}$, is taken exactly 20 minutes after addition of the enzyme mixture and is used to determine the rapidly available glucose (RAG). The second sample, $G_{120}$, is taken exactly 120 minutes after addition of the enzyme mixture. A portion of the $G_{120}$ sample is used to determine the slowly available glucose (SAG). The remainder of said sample is then used to determine the total glucose content in the sample. An interpretation that would better resemble the whole picture of the natural *in vivo* human glucose response is not provided.

Several authors have criticized the Englyst method, for example for providing little predictive value (Garsetti et al., J Am Coll Nutr 2005 24: 441-447) and for incorrect classification of commercial available foods (Brand-Miller et al., European Journal of Clinical Nutrition 2004 58: 700-701). These authors strongly advise against the use of any *in vitro* method for producing GI values for food labeling purposes.

The difficulties of developing a reliable *in vitro* method to predict the *in vivo* glycaemic response to a food are demonstrated by the simple fact that until now only one other example of such a method has been described.

WO 2009/006155, published in January 2009, describes an *in vitro* method for the prediction of the GI of food products. Initially, the fat and protein contents of a food product are determined, whereafter it is ground to a homogenous state. The food product is then digested with a mixture of digestive enzymes for a fixed period of time to provide a digested sample. After treatment of the digested sample to stop enzymatic reactions, amounts of glucose and at least two other monosaccharides (fructose, galactose, lactose, sucrose and/or maltitol) are determined. The *in vivo* GI of the food product is then predicted using a predictive equation or method derived from multivariate analysis with calibration data. The calibration data are obtained from calibration samples with known *in vivo* GI values.

At first glance this method looks promising. However, the *in vitro* method according to WO 2009/006155 also does not mimic the physiological conditions that occur when a food is digested *in vivo.* It seems to be predominantly based on the principles of the Englyst method with only few procedural adaptations. Consequently, it suffers from the same drawbacks as the Englyst method does.

For example, this *in vitro* method requires the food product to be ground to an "essentially homogeneous and finely divided state" which is defined as a degree of grinding equivalent to grinding the food product at temperatures sufficiently low such that the food is a brittle solid. According to WO 2009/006155 such temperatures are generally lower than about -40 °C, preferably below about -78 °C, and more preferably at about temperatures obtainable using liquid nitrogen (-196 °C). Such conditions are not comparable to *in vivo* conditions, e.g. when food is chewed in a mouth having a temperature of around body temperature and wherein said food is mixed with salivary fluid that contains digestive enzymes. Also, the need for such conditions when processing a solid food renders the method time-consuming and laborious. As such steps require additional equipment such as cooling equipment, liquid nitrogen and special grinding mills, costs are added as well. Thus, the *in vitro* method according to WO 2009/006155 seems much less suitable for determining the glycaemic index of solid foods than for determining the glycaemic index of non- or semi-solid foods, where no grinding is involved.

Furthermore, this *in vitro* method comprises a digestive incubation step that is very similar to the incubation step according to the Englyst method. All mixing and digestive steps take place in one vial, and small glass balls are used to mix the ground food with the digestive substances. Also similar to the Englyst method, after addition of HCl and pepsin the ground food is only buffered back to pH at around 5 instead of to at least neutral value (pH = 7-8) as would occur in an *in vivo* intestinal tract, and enzymes for digesting the carbohydrates in the food sample are not added until after the pH has been buffered. Thus, also the *in vitro* method according to WO 2009/006155 does not resemble the physiological environment and involves only one digestive compartment. It again does not provide the typical peristaltic movement of the food from one digestive compartment to another that would occur during *in vivo* digestion of a food.

After exactly 20 minutes ($\pm$ 10 seconds) of digestive incubation, a sample is taken for determination of the glycaemic index of the food product. This sampling procedure is comparable to the procedure for measurement of rapidly available glucose (RAG) according to the Englyst method that also uses a food sample taken after exactly 20 minutes. It only differs in that also data concerning the fat and protein contents of the food product as well as the content of carbohydrates other than glucose are used to determine the glycaemic index of the carbohydrates in the food product. Again, an interpretation that would better resemble the whole picture of the natural *in vivo* human glucose response is not provided.

Thus, the need for an in *vitro* method for predicting the *in vivo* glycaemic response to carbohydrates in a food that is accurate and that resembles *in vivo* physiological conditions remains.

SUMMARY OF THE INVENTION

The current invention provides such a method. Provided is a method for predicting the *in vivo* glycaemic response to carbohydrates in a food wherein the food is digested *in vitro,* **characterized in that** the food is digested in more than one compartment. In one embodiment, said food is digested in two compartments. Also provided is such a method, comprising digesting said food with at least one digestive enzyme selected from the group of amylase, lipase, pepsin, trypsin, chymotrypsin, pancreatin and brush border enzymes. Further provided is such a method, comprising digesting said food with gastric acid. Even further provided is a method for predicting the *in vivo* glycaemic response to the carbohydrates in a food, comprising applying pressure to said food. Preferably, said pressure is applied in a peristaltic manner. More preferably, said pressure is mechanically induced.

Also provided is a method for predicting the *in vivo* glycaemic response to carbohydrates in a food, comprising transferring said food from one compartment to another in a peristaltic motion. In one embodiment, said peristaltic motion is mechanically induced.

Further provided is a method according to the invention, comprising collecting at least one sample from said food before, during, and/or after digestion. Preferably, said method comprises digesting at least one carbohydrate in said at least one sample collected from said food. Also preferred is a method comprising measuring the amount of at least one saccharide selected from the group consisting of glucose, fructose, galactose, lactose and sucrose in said at least one sample collected from said food. Further preferred, said method comprises calculating the *in vitro* glucose or fructose response or both to said food.

Also provided is the use of a method according to the invention for determining the suitability of a food for a subject who would benefit from a normalization of blood glucose levels. Preferably, said subject is suffering from or having increased risk for developing at least one syndrome selected from the group of glucose intolerance, insulin resistance, diabetes mellitus, syndrome X, cardiovascular disease and obesity.

It should be understood that this invention is not limited to the embodiments disclosed in this summary, but it is intended to cover modifications that are within the spirit and scope of the invention, as defined in the claims.

LEGENDS TO THE FIGURES

Figure 1: Calibration data representing the predicted versus observed *in vivo* glycaemic responses to 50 grams of pumpernickel whole kernel rye bread in young volunteers (Juntunen *et al.,* 2002).

Figure 2: Predicted plasma glucose curve (closed dots, n=2) in comparison to the actual *in vivo* plasma glucose curve (open triangles, n=10, mean $\pm$ SEM) after the consumption of Durum wheat semolina pasta. Dotted lines indicate $\pm$ 5% deviation from the actual *in vivo* curve.

Figure 3: Predicted plasma glucose curve (closed dots, n=2) in comparison to the *in vivo* plasma glucose curve (open triangles, n=9, mean $\pm$ SEM) after the consumption of Parboiled pearled wheat. Dotted lines indicate $\pm$ 5% deviation from the *in vivo* curve.

Figure 4: Predicted plasma glucose curve (closed dots, n=2) in comparison to the *in vivo* plasma glucose curve (open triangles, n=6, mean $\pm$ SEM) after the consumption of Kellogg's Special K. Dotted lines indicate $\pm$ 5%

deviation from the in *vivo* curve.

Figure 5: Predicted plasma glucose curve (closed dots, n=2) in comparison to the *in vivo* plasma glucose curve (open triangles, n=10, mean $\pm$ SEM) after the consumption of Nutrient formula A. Dotted lines indicate $\pm$ 5% deviation from the *in vivo* curve.

DETAILED DESCRIPTION OF THE INVENTION

Definitions

The term "digestion" as used herein is defined as any process or method by which a food is prepared for absorption into the blood. This includes *in vivo* and *in vitro* degradation of a food. It comprises any process or method for preparing a food for consumption, such as cooking, baking or frying, any method or process for crushing a food such as chewing or grinding, any method or process for degrading the macromolecules in a food such as enzymatic digestion or acidic hydrolysis, any method or process for mixing a food with digestive compounds, such as peristalsis or application of mechanical pressure and any method or process for transporting a food through a gastrointestinal tract or from one compartment to another, such as peristaltic movement.

As used herein, "carbohydrate" means any compound of general structure $(CH_2O)n$, wherein n is a positive integer and $n \geq 3$, that can be digested by a human, animal, bacterium or fungus, including yeast. This includes, but is not limited to, all mono-, di-, oligo- and polysaccharides.

The term "monosaccharides" as used herein means any compound of general structure $(CH_2O)n$, wherein n is a positive integer and $3 \leq n \leq 9$, that can be produced in and possibly absorbedfrom the gastrointestinal tract of a human or an animal. This includes, but is not limited to, glucose, fructose, galactose, whether or not hydrogenated or otherwise modified, including but not limiting to sorbitol, mannitol and xylitol.

The term "digestive enzyme" as used herein means any enzyme, purified as well as in (natural) mixtures, that breaks down or modifies di-, oligo- or polymeric macromolecules into their smaller building blocks or other molecule structures and that is of human, animal, bacterial or fungal origin, or produced by biotechnological processes. It includes any enzyme that breaks down or modifies carbohydrates such as, but not limited to, ptyalin, $\alpha$-amylase or $\beta$-amylase, isomaltase, maltase, sucrase and lactase. It also includes any enzyme that breaks down or modifies proteins or peptides such as, but not limited to, pepsin, trypsin, chymotrypsin, peptidase, carboxypeptidase, peptase, elastase, gelatinase and nuclease. Furthermore, it includes any enzyme that breaks down or modifies fat such as, but not limited to, lipase, steapsin and pancreatin.

As used herein, the term "brush border enzymes" is defined as a mixture comprising at least one of the digestive enzymes that are located in the layer of the brush border (microvilli) on intestinal epithelial cells of humans and animals such as, but not limited to, aminopeptidases, glucoamylase (also known in the art as maltase), sucrase (also known in the art as sucrase-isomaltase) and lactase (also known as galactosidase).

The term "compartment" as used herein is defined as any space that comprises at least one wall and is suitable for containing a food and digestive enzymes. Thus, the term includes any such space wherein the at least one wall is made from a rigid material such as a glass or a metal, and any such space wherein the at least one wall is made from a non-rigid material such as rubber. It also includes any such space further comprising an inner wall or structure made from a non-rigid material, such as a plastic layer or a rubber hose. It further includes any such space being open at at least one end, and any such space comprising one or more connecting passages. Finally, the term includes any such space comprising two or more subspaces that each comprise at least one wall, are suitable for containing a food and digestive enzymes and that are connected to each other by means of at least one connecting passage. Non-limitative examples of a compartment according to the definition are a glass cup, a metal beaker, a plastic vial, a rubber tube, a rubber sac and a tube that comprises an inner hose made of rubber and that is connected to another compartment.

As used herein, "peristalsis" means any series of successive muscle contractions passing along the walls of a hollow muscular structure (such as the oesophagus, stomach or intestines), applying pressure to the contents of that structure and forcing them onward or backward.

The term "peristaltic movement" as used herein is defined as any *in vitro* movement of the content in a compartment, including a food, that is induced by any process resembling peristalsis. This includes any *in vitro* movement of the content and/or a food within a compartment and any *in vitro* movement from a food from one compartment to another.

Description

The inventors surprisingly found that the accuracy of predicting the *in vivo* glycaemic response to a food is increased when said food is digested in more than one compartment. Provided is a method for predicting the *in vivo* glycaemic response to carbohydrates in a food wherein the food is digested *in vitro,* **characterized in that** the food is digested in more than one compartment. The method can be used to predict the *in vivo* glycaemic response to any type of food. Such food may be a solid food, a semi-solid food or a non-solid food. A "food" as used herein can be a single food component, including a liquid or a solid food, and it may be a food matrix made up of a mixture of single food components up to a complete meal.

The number of compartments may vary depending on the type of food to be tested. For example, digestion of a liquid food may require fewer compartments than digestion of a solid food. In one embodiment, the invention provides a method for predicting the *in vivo* glycaemic response to carbohydrates in a food wherein the food is digested *in vitro,* **characterized in that** the food is digested in two compartments.

The number of compartments may also depend on the type of digestion that is required. It may for instance be required that the digestion resembles the digestion by humans or animals. Animal digestion can be of different types, such as digestion by a carnivore, an omnivore or a herbivore. For example, a digestion may resemble human, mammalian or avian digestion. More specifically a digestion may resemble human, digestion or digestion of an animal selected from, but not limited to, the group consisting of pig, horse, dog, cat, mouse, rat, cow, sheep, chicken and ostrich. Therefore the method may comprise digesting a food in three or more compartments.

It may thus be required that the digestion resembles the typical digestion by a ruminant such as a cow or a deer. Thus, in yet another embodiment the method comprises digesting a food in more than three compartments. For example, a food is digested with amylase in one compartment, digested with gastric acid in another compartment, digested with trypsin and lipase in a further compartment and then fermented with bacteria in yet a further compartment. Thus, a skilled person will appreciate that the appropriate number of compartments for digesting a food can be readily determined, depending on the type of food and the type of digestion.

It may be clear from the above that it is preferred that the conditions of an *in vitro* method for predicting the glycaemic response to a food mimic the *in vivo* physiological conditions wherever possible. For example, each digestion step of the food should preferably be conducted at a temperature that reflects the average body temperature, i.e. a temperature between 35 °C and 39 °C, preferably around 37 °C. Thus, not only the temperature of the food to be digested, but also the temperature of the digestion compartments and of the solutions that are added at each compartment should preferably deviate as less as possible from the average body temperature. However, it will be well understood by a person skilled in the art that exceptions may occur. For example, a food may be cooked, baked or prepared otherwise according to instructions before digestion and thus may have a temperature that is above or below body temperature. Nevertheless, it should be avoided to digest food having a temperature of more than about 70 °C or lower than about -20 °C.

A food is typically digested by at least one digestive enzyme. A person skilled in the art will appreciate which enzymes are best suited for the *in vitro* methods according to the invention to digest a certain type of food. For example, enzymes such as salivary and pancreatic amylase, and brush border enzymes such as sucrase and lactase, are best suited for digesting the carbohydrates in a food. Other enzymes such as pepsin, trypsin, chymotrypsin, and lipase may also be used, to free the carbohydrates from the food matrix. To reflect *in vivo* physiological conditions such digestive enzymes are preferably selected from enzymes of human or animal origin, such as amylase that is found in human and animal salivary fluid.

Therefore, in one aspect the invention provides a method for predicting the *in vivo* glycaemic response to carbohydrates in a food wherein the food is digested *in vitro,* **characterized in that** the food is digested in more than one compartment, comprising digesting said food with at least one digestive enzyme selected from the group of amylase, lipase, pepsin, trypsin, chymotrypsin, pancreatin and brush border enzymes. For example, a food is digested with amylase and pepsin in one compartment and digested with lipase and pancreatin in another compartment. The method may also comprise digesting a food with amylase in one compartment, digesting said food with pepsin in another compartment and digesting said food with pancreatin in yet another compartment.

It will be clear that the digestion of a food can be supported by substances other than digestive enzymes, such as gastric acid and bile. Therefore, in another embodiment the method according to the invention comprises digesting a food with gastric acid. For example, a food is digested with amylase in one compartment and then digested with gastric acid in another compartment. Also other substances are secreted in the digestive tract. For example, potassium chloride and sodium chloride are secreted during *in vivo* digestion in the stomach. When the acidified food leaves the stomach and arrives in the duodenum, sodium bicarbonate is secreted to neutralize the acidic gastric juice. Thus, such substances may also be readily applied in the methods according to the invention, as will be appreciated by a person skilled in the art.

During *in vivo* digestion of a food, a food is typically kneaded throughout the whole gastrointestinal tract. This kneading starts in the oral cavity, where pressure is usually applied to the food by chewing. The purpose of chewing a food is to physically break it into smaller pieces and disrupt its structure. During chewing the food is also mixed with salivary fluid that contains digestive enzymes such as amylase. When the food has been sufficiently chewed, the food is swallowed and enters the oesophagus. The food is now in an "as eaten" state.

It will be clear to a skilled person that when using a method according to the invention to digest a food, it is preferred that the food is or becomes in its "as eaten" state. Not only may this require that the food is processed before digestion, e.g. by cooking it as specified, but also that the food is physically disrupted by a process that mimics chewing.

Therefore, in another aspect the invention provides a method for predicting the in *vivo* glycaemic response to carbohydrates in a food wherein the food is digested *in vitro* in more than one compartment, comprising applying pressure to said food. Such pressure may be applied by any suitable means. For example, a food may be broken or divided into small pieces by hand force. Also, a food may be ground with a mortar or a food processor.

As mentioned above, the conditions under which the pressure is applied should resemble physiological conditions wherever possible. For example, when grinding a food with a mortar or food processor care should be taken to prevent the temperatures of both the food and the mortar or food processor from deviating largely from body temperature. A skilled person will also appreciate that the intensity and duration of applying pressure to a food to obtain its "as eaten" state depend on the type of the food. For example, less pressure may be required when digesting a semi-solid or non-solid food. It may even be sufficient to not apply any pressure at all, for example when a liquid food such as lemonade or an energy drink is digested. When digesting a solid food such as pumpernickel bread or a lollipop, more pressure will be required.

When the food *in vivo* has been swallowed and has entered the oesophagus, peristalsis enables further transportation of the food from one digestive compartment to another. Also, peristalsis enhances mixing of the food with the digestive enzymes to improve digestion. Thus, to resemble *in vivo* conditions pressure is preferably applied *in vitro* to a food in a peristaltic manner. Under *in vitro* conditions, peristalsis may be mimicked by mechanical force. Therefore, a method according to the invention may comprise applying pressure to a food, wherein said pressure is applied in a peristaltic manner and wherein said pressure is mechanically induced.

Suitable means for mechanically inducing pressure to a food, preferably in a peristaltic manner, will be known by a person skilled in the art. Said pressure may be induced by pressure chambers that compress a gas or a liquid surrounding a compartment. For example, US 5,525,305 and EP 0 642 382 B1 describe an *in vitro* model of a gastrointestinal tract comprising a reactor system. This reactor system comprises at least one unit consisting of two or more pressure chambers. A flexible hose is situated in each pressure chamber. This hose is fixed in such a way that a gas or a liquid can either be discharged or exhausted from the spaces between the wall of the pressure chamber and the hose. The discharged gas or liquid induces pressure to the hose, thus inducing pressure to the contents of the hose such as a food. This pressure can be decreased again by exhausting the gas or liquid. The amount of gas or liquid that is discharged or exhausted is controlled by a control means. In this way a pressure can be applied that resembles peristalsis. Thus, the reactor system provides a suitable *in vitro* method for kneading a food. Furthermore, when using more than one unit multiple pressure chambers can be put into action. By connecting these pressure chambers in a sequential manner also a suitable *in vitro* method for transporting a food is provided. Thus, a reactor system as described in US 5,525,305 and EP 0 642 382 B1 is very suitable for use in the *in vitro* methods according to the invention.

It will be appreciated by a person skilled in the art that the method according to the invention can be used to simulate any type of digestive tract, especially when means for applying peristaltic pressure are applied and the compartments are connected to each other in a sequential manner so that a food can be transferred from one compartment to another in a peristaltic motion. Therefore, in another embodiment the method according to the invention comprises transferring a food from one compartment to another in a peristaltic motion. For example, a food is digested with amylase in one compartment, then transferred in a peristaltic motion to another compartment wherein the food is further digested with gastric acid, then transferred in a peristaltic motion to another compartment wherein the food is even further digested with pancreatin, lipase and bile.

Under *in vivo* conditions the duration of transferring a food from one compartment to another depends on the type of food that is digested. For example, *in vivo* it takes about 30 minutes to transfer half of the contents of the stomach to the duodenum when a liquid food is digested, and about 40 minutes if a solid food is digested. Therefore, in one embodiment the *in vitro* method according to the invention comprises transferring a food in a peristaltic motion from a first compartment to a second compartment, according to a controlled transfer curve wherein half of the contents of said first compartment have been transferred to said second compartment in 10 to 60 minutes, preferably in 25 to 45 minutes. For example, a solid food is digested with amylase in a first compartment, gradually transferred in a peristaltic motion from the first compartment to a second compartment, digested with gastric acid and pepsin in the second compartment and then gradually transferred in a peristaltic motion to a third compartment, with a transfer time wherein half of the contents of the second compartment have been transferred to the third compartment in 40 minutes.

It will be clear to a skilled person that the peristaltic motion is ongoing. Thus, when half the contents of a compartment have been transferred by peristaltic motion from that compartment to another compartment, the peristaltic motion continues at least until almost all of the remaining part of the contents also has been transferred to the other compartment. The passage from one compartment to the following compartment can not only be set according to a linear line, but also to an algorithmic curve, for example to simulate realistically the emptying of food from the stomach of humans or a specific animal species related to the type of food..

Also the duration of the *in vivo* digestion of a food in the intestinal tract depends on the type of food that is digested. Typically, in humans it takes between 2 and 6 hours for a food to pass through the small intestines and to enter the colon. Therefore, in one embodiment the method according to the invention comprises transferring a food from one compartment to a further compartment in a peristaltic motion, wherein the duration of digesting said food in said further compartment is between 60 and 360 minutes, preferably between 110 and 270 minutes. For example, a food is digested with amylase in a first compartment, transferred in a peristaltic motion to a second compartment, digested therein with pepsin, then transferred in a peristaltic motion to a third compartment wherein half the contents of the second compartment have been transferred to the third compartment in 40 minutes, and then digested therein with lipase and pancreatin during 180 minutes.

In order to calculate the *in vitro* glycaemic response to carbohydrates in a food, at least one sample needs to be collected from that food. Therefore, in yet another aspect the invention provides a method for predicting the *in vivo* glycaemic response to carbohydrates in a food wherein the food is digested *in vitro,* **characterized in that** the food is digested in more than one compartment, comprising collecting at least one sample from said food during and/or after digestion.

As mentioned above, in the art a food's GI is calculated based on the *in vivo* blood glucose response to the food. The *in vivo* blood glucose response is typically measured over a total period of 120 minutes, and is characterized by the presence or absence of a peak in the blood glucose level. Both the presence and shape of the peak depend on the type of carbohydrates in the food. For example, simple carbohydrates such as glucose are rapidly digested and absorbed, inducing a fast and relatively large increase in blood glucose values. The resulting blood glucose response curve has a typical shape, characterized by a rapidly occurring, high peak that has a sharp form such as a "spike". If a food contains more complex carbohydrates that need more digestion time, a more gradual increase in blood glucose levels will be observed. The blood glucose response curve will then display a rise that is much more gradual over time, sometimes without an obvious observable peak.

Thus, it will be appreciated by a person skilled in the art that preferably a number of samples is taken from the *in vitro* digested food such that an *in vitro* blood glucose response curve can be obtained that adequately mirrors its *in vivo* blood glucose response. For example, samples are collected at regular 10 minute intervals during *in vitro* digestion of a food. The schedule of sampling may also be of a more irregular nature. Samples may for instance be taken every 5 minutes during the first hour of *in vitro* digestion, and every 15 minutes during the second hour of *in vitro* digestion.

A person skilled in the art will also appreciate that proper procedures for collecting food samples *in vitro* must be applied, to avoid sampling errors. For example, aliquots of the digested food may be taken and filtered or dialyzed to remove any solid matter or sediment before collecting a sample from each aliquot. Alternatively, dialysates or filtrates of the food may be obtained at more than one time point during *in vitro* digestion of the food, and a sample may be collected from each obtained dialysate or filtrate. It will be clear that any *in vitro* sampling procedure should be standardized for all food samples to be collected in order to obtain representative fractions of the digested carbohydrates.

The final step of *in vivo* digestion of dietary carbohydrates into monosaccharides such as glucose and fructose is conducted by brush border enzymes. When this final digestion step is completed, the resulting monosaccharides are transported into the epithelial cells. From there, the monosaccharides enter the bloodstream and are transported to the liver for further processing. Thus, a person skilled in the art will appreciate that the carbohydrates in samples collected from an *in vitro* digested food also need further digestion. Therefore, in one embodiment the method according to the invention comprises further *in vitro* digestion of at least one carbohydrate in at least one sample collected from an *in vitro* digested food.

Such further *in vitro* digestion preferably mimics the *in vivo* digestion by the brush border enzymes. Thus, the further *in vitro* digestion is preferably performed by incubating the food samples collected from the earlier *in vitro* digestion step with brush border enzymes. For example, a food is digested *in vitro* with amylase in one compartment, transferred in a peristaltic motion to another compartment, digested therein with gastric acid, then transferred to a further compartment wherein food samples are collected at regular 10 minute intervals, and where after the collected food samples are incubated *in vitro* with brush border enzymes.

It is preferred that the further *in vitro* digestion is performed under conditions that resemble *in vivo* conditions. For example, under *in vivo* conditions it takes some time for the brush border enzymes to further digest the carbohydrates into monosaccharides, depending on the amount and type of carbohydrates in said food. The further *in vitro* digesting is preferably conducted during a period of between 2 and 24 hours, preferably between 16 and 20 hours (overnight). For example, samples that have been collected from an *in vitro* digested food are further digested *in vitro* by incubation with brush border enzymes for 20 hours.

During further *in vitro* digestion, additional compounds may be added to the collected samples. For instance, one or more compounds may be added that prevent potential microbial growth. Such growth would deplete the glucose concentration in the samples. Any method known in the art can be used to prevent microbial growth in the collected samples, and can be applied by a person skilled in the art. For example, collected samples may be preserved with sodium azide. Also, a skilled person will appreciate the amount of azide to be preventively added. The amount of sodium azide added to the sample may be between 0.01% w/v and 1% w/v, preferably about 0.05% w/v. To prevent microbial growth, also ultrafiltration of the sample may be used.

Although somewhat less preferable, further *in vitro* digestion of the carbohydrates in a collected food sample may also be conducted by non-enzymatic digestion methods. Such methods are known by a person skilled in the art. For example, it is well known in the art that carbohydrates such as sucrose, maltose and glycogen can be hydrolyzed into their monosaccharides glucose and fructose by acid treatment. Thus, acids are suitable compounds when conducting non-enzymatic hydrolysis of carbohydrates. Therefore, in yet another embodiment the method comprises hydrolyzing at least one carbohydrate in at least one sample collected from a food by an acid. Examples of suitable acids include, but are not limited to, nitric acid, phosphoric acid, sulphuric acid and hydrochloric acid.

As a skilled person will understand, the monosaccharide contents in samples collected from a food may be measured by any means of quantitative analysis that is suitable to measure monosaccharides. For example, the monosaccharide contents can be measured using high performance liquid chromatography (HPLC), high performance ion chromatography (HPIC) or enzymatic analysis, for example a conventional glucose assay or a glucose-fructose assay. These examples of suitable methods for quantitative analysis are not meant to be limitative.

A skilled person will also understand that when using such methods the amount of any monosaccharide can be measured that provides data for determining the *in vitro* glucose response to a food. For example, the amounts of glucose, fructose and galactose can be measured in the samples collected from a food that was digested *in vitro* according to the methods of the invention. The measured amounts represent the level of (mono)saccharides at each sampled time point, and can be used to plot the *in vitro* glucose response curve to that food. The *in vivo* glycaemic response or GI of the food can then be predicted by comparing the AUC of the *in vitro* glucose response curve to the AUC of the *in vivo* glucose response of a reference food.

Therefore, in one embodiment the method comprises measuring the amount of glucose, whether or not in combination with fructose and galactose, in at least one sample collected from a food. For example, a food is digested *in vitro* with gastric acid in one compartment, transferred in a peristaltic motion to another compartment and then digested therein with digestive enzymes. During the enzyme digestion of the food, samples are collected at regular 5 minute intervals. These samples are then further digested *in vitro* for 12 hours with brush border enzymes. Finally, the amounts of glucose and fructose in the further digested food samples are determined by for instance a conventional assay.

Once the monosaccharide contents of the samples collected from a food have been measured the *in vitro* glucose response to the food can be calculated, as mentioned above. Thus, in a further embodiment the method comprises calculating the *in vitro* glucose response to said food.

Based on the *in vitro* glucose and fructose response as result of the *in vitro* digestion steps mentioned above, the *in vivo* glycaemic response can be predicted. The *in vitro* glucose and fructose response curves represent the amounts of glucose and fructose that are digested and available for absorption from the intestine to enter the body, In healthy persons the intestinal absorption of glucose and partly of fructose gives an instant reaction in the release of insulin by the pancreas into the blood. This insulin is responsible for lowering the blood glucose concentration after intestinal absorption of glucose and fructose. This means that for the prediction of *in vivo* blood glucose levels (glycaemic response), based on measured *in vitro* glucose and fructose response curves, the physiological processes in the body should be taken into consideration.

For this purpose, a kinetic (*in silico*) model describing these physiological processes involved in glucose homeostasis is used to predict the *in vivo* glycaemic response to a food based on the obtained *in vitro* glucose response to that food. This kinetic model is based on the homeostatic model assessment (HOMA) of Wallace et al. (Diabetes Care 2004 vol. 27(6): p. 1487-1495) which can be used to assess β-cell functioning and insulin resistance from baseline (fasting) glucose and insulin concentrations. The HOMA model is mainly based on experimental data collected in human studies and is suitable to predict glucose and insulin concentrations of both diabetic and non-diabetic individuals. This *in silico* model enables a translation of experimental *in vitro* glucose data to *in vivo* plasma glucose profiles. In addition, a glycaemic response that would occur *in vivo* after ingesting of a certain food product can be predicted.

As mentioned above, foods with a low GI (i.e., foods that elicit a small glycaemic response) are considered to provide significant health benefits in general, especially for subjects suffering from or having increased risk for developing syndromes such as glucose intolerance, insulin resistance, diabetes mellitus, syndrome X or metabolic syndrome, cardiovascular disease and obesity. Foods with a high GI (i.e., foods that elicit a large glycaemic response) may also provide benefits. For example, they are suitable for a person in need of energy recovery after endurance exercise or for a person suffering from diabetes mellitus who is experiencing hypoglycemia.

In a further aspect, the invention therefore provides the use of any method according to the invention for determining the suitability of a food for a subject who would benefit from a normalization of blood glucose levels. In a preferred embodiment, said subject is suffering from or having increased risk for developing at least one syndrome selected from the group of glucose intolerance, insulin resistance, diabetes mellitus, syndrome X, cardiovascular disease and obesity.

The invention is exemplified by the following examples. These examples are intended to illustrate the invention, without limiting the scope thereof in any way.

EXAMPLE 1

This example describes the *in vitro* digestion of different types of foods by a method according to the invention. Also the calibration of the *in vitro* method is described. Furthermore, the accurate prediction of *in vivo* glycaemic index (GI) values for these foods is demonstrated by comparison of these predicted values to known GI values of reference products.

*In vitro* digestion of test foods

Four different test foods (2 liquid foods, 2 solid foods) were digested *in vitro* according to the invention As shown in Table 1, for each test food the amount of available carbohydrates (CHO) was standardized.

Table 1. Overview of four different foods tested in the *in vitro* method

| Test food | Manufacturer | Intake of test food in *in vitro* model | Amount of available CHO *in vivo**  |
|---|---|---|---|
| 1. Pure glucose | Sigma | 6.25 g | 6.25 g |
| 2. Drink Formula (Diasip) | Nutricia | 35.5 g | 6.25 g |
| 3. White bread (Bolletjes Toast) | Bolletje | 6.25 g | 6.25 g |
| 4. Pumpernickel whole kernel rye bread | WEPU Brot | 13.5 g | 6.25 g |
| * Data for amount of available carbohydrates (CHO) are based on the information on the package of the products. | | | |

First, each food was processed to an "as eaten" state by processing the food according to instructions provided by its manufacturer and grinding the food into small pieces when necessary. Each food was then individually digested *in vitro* in duplicate in a two compartment system representing the stomach and small intestine.

For each food the quantity as specified in Table 1 was mixed with artificial saliva, containing 6.2 g/l sodium chloride, 2.2 g/l potassium chloride and 0.3 g/l calcium chloride (for product 1, 57 ml; for product 2, 48 ml; for product 3, 57 ml, for product 4, 44 ml) and drinking water (57 ml; 37 ml; 57 ml; and 63 ml for products 1-4, respectively). The pH of the food-saliva-water mix was set at pH 5.0 and warmed at 30°C $\pm$ 2 °C. The digestion started by the addition of 5.5 mg $\alpha$-amylase to the food-saliva-water mix and incubation for 1 min. The food-saliva-water-mixture was then immediately introduced into the gastric compartment (pre-warmed to 37 °C $\pm$ 2 °C) of a Tiny-TIM system (TNO gastro-Intestinal Model, TNO, the Netherlands), containing 5 ml gastric juice at pH 2. The total volume in the stomach compartment at the start was 125 ml.

During the digestion experiment in the Tiny-TIM system, gastric acid (Merck Hydrochloric acid, 1Mol/L) was added at 0.25 ml/min to the stomach compartment, alternating with water, to control the pH curve in the stomach. Further gastric juice was added at 0.25 ml/min, containing electrolytes, gastric lipase (18 units/ml Rhizopus lipase F-AP 15, Amano Pharmaceuticals) and, pepsin (300 units/ml Sigma P7012). During digestion, the food mix was transferred from the gastric compartment to a small-intestinal compartment in a peristaltic motion. The half time of gastric emptying was set at 40 minutes for solid products and at 30 minutes for liquid products. In the small-intestinal compartment the pH was adjusted to 7.0 $\pm$ 0.2 by the addition of sodium-bicarbonate. Further, pig bile (0.25ml/min), pancreatic juice (0.125 ml/min) and small-intestinal electrolytes (0.125 ml/min) were added to this compartment. The available carbohydrates were then absorbed from the food mix through a membrane unit, kept at 37 °C, that was connected to the small-intestinal compartment. From the resulting dialysis stream, 1.8 ml samples were continuously collected in duplicate by a fraction collector (Gilson 203B, Gilson Inc., Middleton, USA), at 10 minute intervals for 180 minutes. The collected samples were stored in duplicate 96 deep-well plates. One plate was used for direct analysis. The duplicate plate was sealed and stored at - 20 °C or lower.

Plated samples, as well as reference solutions of maltose and sucrose, were further digested *in vitro* by incubation with brush border enzymes. To this aim, samples and reference solutions were diluted with purified water to concentrations suitable for efficient further digestion. Than, a 1.0 M citrate buffer at pH 6.0 (50 $\mu$l) and a brush border enzyme mix were added to each sample and reference solution. The brush border enzyme mix (100 $\mu$L) was freshly prepared from Sigma intestinal acetone powders according to instructions (Sigma charge 024K7005) and the addition of lactase (F Amano-50; 1,0 mg/ml). In order to eliminate microbial growth the enzyme solution was ultra-filtered over 0.2 $\mu$m cellulose acetate. Digestion of the different monosaccharide components in the samples was carried out at 37 °C over night (20 hours).

Calibration of the predictive model and prediction of *in vivo* glycaemic responses and GI values

Aliquots (0.25 ml; diluted sample with water to an expected glucose concentration not exceeding 0.15% w/v or 1.5 mg/ml) of the digested samples and reference solutions and aliquots of glucose and fructose standard solutions were consequently transferred into micro-titer plates for analysis, using an enzymatic glucose-fructose assay (R-Biopharm AG, Darmstadt, Germany) according to instructions. The concentrations of D-glucose and D-fructose were determined at 340 nm. Obtained glucose and fructose concentrations were multiplied with the dialyzed volume of the Tiny-TIM *in vitro* system per 10 min. interval to obtain the absolute amount of glucose and fructose per 10 min sampling period.

The obtained *in vitro* glucose and fructose data were extrapolated to predicted *in vivo* plasma glucose level profiles by using an *in silico* model which is based on the HOMA model of Wallace et al. (Diabetes Care 2004 vol. 27 (6): p. 1487-1495). This *in silico* model (Version 2.7) was described using Microsoft® Visual Basic® Macro integrated in Microsoft® Excel® spreadsheets. Model performance was assessed by calculating the average relative deviation of the predicted and *in vivo* observed glycaemic response curve from 0 to 180 minutes:

$$\{\Sigma \mid 0\text{-}180 \mid ( ( \mid \text{CGluc Pred} - \text{Cgluc Obs} \mid )/\text{CGluc Obs} ) \}/n$$

The *in silico* model was calibrated using a dataset from Juntunen et al. (Am. J. Clin. Nutr. 2002 vol. 75: p. 254-262). In that study, a serving of whole kernel rye bread (pumpernickel) with an available carbohydrate load of 50 grams was consumed by young volunteers.

Importantly, a time shift of 10 minutes was used to account for the difference between Tiny-TIM and the onset of glucose absorption in humans. A number of parameters of the *in silico* model were fitted using constraints on between-organ glucose fluxes in the model and the observed glucose and insulin plasma levels. The final *in silico* parameters as used in the final version of the *in silico* model are shown in Table 2. Using these values, the glycaemic response of the model was set to reflect the response of the human volunteer population that participated in the Juntunen study, with an average age of 30 years and a Body Mass Index (BMI) of around 20 kg/m$^2$.

Table 2. Final calibrated settings for the HOMA *in silico* model, version 2.7.

| HOMA parameters for young adults (30 years of age) | |
| --- | --- |
| VD glucose (I) | 14.00 |
| VD insulin (I) | 13.00 |
| Fasting glucose level (mmol/l) | 5.03 |
| Baseline insulin level (pmol/l) | 45 |
| Insulin degradation (KINS) | 0.17 |
| Liver sensitivity for glucose (LA1) | -2.50 |
| Liver sensitivity for insulin (LA2) | -2.20 |
| Beta Vmax | 900.00 |
| Beta IC$_{50}$ | 7.50 |
| Beta A1 | 5.00 |
| MVMAX | 2.60 |
| MKM | 0.01 |
| MIC$_{50}$ | 80.00 |
| MA1 | 1.50 |
| MA2 | 2.00 |
| Iabase | 6.7 |

The glucose and fructose concentration curves in the *in vitro* dialysates of the test foods, as measured by enzymatic assay, were used as input in the *in silico* model to predict the human *in vivo* plasma glucose and insulin responses in healthy subjects. For the *in vitro* fructose concentration it was calculated that it contributed to the glycaemic response for 20% in comparison to the *in vitro* glucose concentration. To this aim, the predicted *in vivo* blood glucose curves were compared to actual *in vivo* glycaemic response curves observed in humans after intake of similar products (Lee et al., Eur J Clin Nutr 1998 vol. 52: 924-928, Hofman et al., Eur J Clin Nutr 2004 vol. 58: 1553-1556, Hofman et a/., Asia Pac J Clin Nutr 2006 vol. 15(3): 412-417, Juntunen et al., Am J Clin Nutr 2002 vol. 75: 254-262, Foster-Powell et al., Am J CLin Nutr 2002 vol. 76(1): 5-56 and Liljeberg et al., Eur J Clin Nutr 1992 vol. 46(8): 561-575).

When data from literature were given as plasma glucose concentration corrected for baseline, the data were adjusted back using a fasted plasma glucose concentration of 5.03 mmol/l (Genuth et al., Diabetes Care 2003 vol. 26: 3160-3167). Also a preliminary similarity criterion was used. Similarity was considered when the peak of the obtained *in vitro* glycaemic curve deviated no more than 10% of its maximum value and not more than 10-15 minutes in appearance from the *in vivo* curve. Finally, also the GI of the test foods was calculated, based on the AUC for the predicted plasma glucose curves.

Results and conclusion

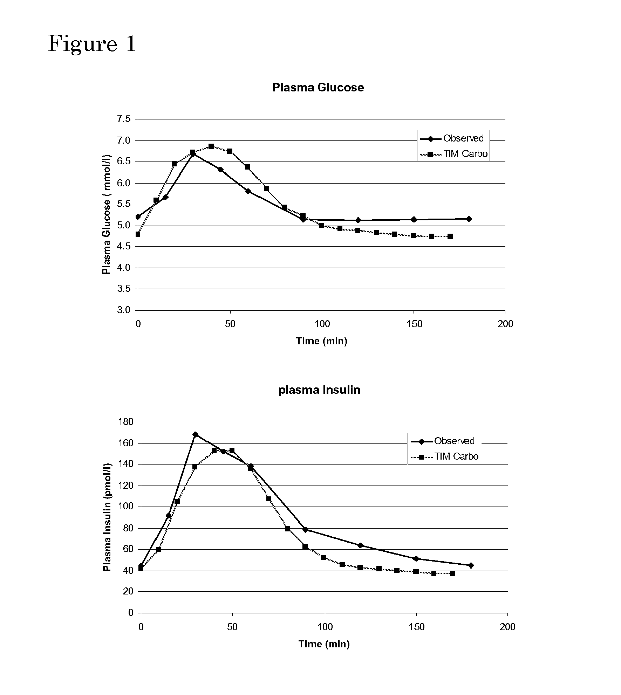

Figure 1 shows the prediction by the *in silico* model of the in *vivo* glucose and insulin responses to a 50 grams pumpernickel meal in young volunteers, based on the calibration data of the Juntunen study. Analysis revealed that the predicted glucose response deviated no more than 6.7% from the actual *in vivo* response observed by Juntunen *et al.*

Table 3 shows the predicted and actual *in vivo* maximum glucose concentrations ($C_{max}$) and the predicted incremental areas under the curve (iAUC) of the test foods. It can be observed that each predicted $C_{max}$ closely resembles the actual $C_{max}$ that was observed *in vivo.* Also shown are the GI values of the test foods, as calculated by the *in silico* model based on their iAUC. The GI of pure glucose was calculated at 100, the GI of white bread at 74.

Thus, as demonstrated by Figure 1 and Table 3, the *in vitro* methods according to the invention reliably predict the *in vivo* GI and glycaemic response to both solid and non-solid foods.

Table 3. Predicted and actual *in vivo* $C_{max}$ and predicted iAUC of the test foods. Also shown are the calculated GI values of the test foods, based on their predicted iAUC and related to white bread and glucose as reference foods.

| Test food | Predicted $C_{max}$ (mmol/l) | Actual $C_{max}$ (mmol/l) | Predicted iAUC | GI (white bread as ref.) | GI (glucose as ref.) |
|---|---|---|---|---|---|
| Pure glucose | 8.35 | 8.62 | 127.91 | 136 | 100 |
| Drink formula | 5.68 | 5.48 | 9.36 | 10 | 7 |
| White bread | 6.9 | 6.9 | 94.16 | 100 | 74 |
| Pumpernickel | 6.9 | 6.7 | 95.51 | 101 | 75 |

EXAMPLE 2

This example describes a further experiment, wherein *in vivo* glycaemic responses were predicted for multiple solid and non-solid test foods based on the *in vitro* method according to Example 1. For each test food, the predicted *in vivo* response was again compared to its actual *in vivo* glycaemic response as observed in human study populations. The actual *in vivo* responses were provided by the manufacturers of the test foods, together with demographical data describing the human study populations.

Table 4 lists the different food products that were tested in this experiment. For each type of test food the amount of available carbohydrates (CHO) was standardized.

Table 4. Overview of foods tested in the *in vitro* method. CHO = carbohydrates, HM = High Maize

| Test food | Manufacturer | Intake of test food in *in vitro* model | Amount of available CHO *in vivo** |
|---|---|---|---|
| Durum wheat semolina pasta (Spaghetti Integrali) | Barilla | 9.3 g | 50.0 g |
| Parboiled pearled wheat | Barilla | 9.61 g | 50.0 g |
| Legume pasta (Spaghetti Plus) | Barilla | 10.33 g | 50.0 g |
| Soft bread | Barilla | 13.24 g | 50.0 g |
| High-fibre biscuit | Barilla | 11.04 g | 50.0 g |

(continued)

| Test food | Manufacturer | Intake of test food in *in vitro* model | Amount of available CHO *in vivo**  |
|---|---|---|---|
| Breakfast Rusk | Barilla | 8.23 g | 50.0 g |
| Nutrient formula A | Abbott | 49.45 g | 25.0 g |
| Nutrient formula B | Abbott | 39.66 g | 50.0 g |
| Breakfast cereals (All Bran) | Kellogg | 14.9 g | |
| Breakfast cereals (Special K) | Kellogg | 7.6 g | |
| soluble fibre A | Tate & Lyle | 3.13 g | 25 g |
| soluble fibre B | Tate & Lyle | 3.13 g | 25 g |
| soluble fibre E | Tate & Lyle | 3.13 g | 25 g |
| insoluble fibre C | Tate & Lyle | 3.13 g | 25 g |
| insoluble fibre D | Tate & Lyle | 3.13 g | 25 g |
| Control bread | Nat. Starch | 8.9 g | 36 g |
| HM260 bread dose 1 | Nat. Starch | 9.0 g | 29.4 g |
| HM260 bread dose 2 | Nat. Starch | 9.1 g | 28.0 g |

* Data for amounts of available carbohydrates (CHO) were provided by the food's manufacturers.

Table 5 displays the demographical data of the human study populations for each tested food, including gender, age and body mass index (BMI). The number of study subjects ranged from 6 to 22 subjects per population.

Table 5. Demographical data of the human study groups. BMI = Body Mass Index (kg/m$^2$), HM = High Maize, m = male, f = female.

| Food | Number of study subjects | Gender | Age (years) | BMI (kg/m$^2$) |
|---|---|---|---|---|
| Durum wheat semolina pasta (Spaghetti Integrali) | 10 | 3 female, 7 male | 26.5±2.5* | 21.9±0.9* |
| Parboiled pearled wheat | 9 | 7 female, 6 male | 26.2±1.8* | 21.6±0.7* |
| Legume pasta (Spaghetti Plus) | 10 | 3 female, 7 male | 26.5±2.5* | 21.9±0.9* |
| Soft bread | 10 | 3 female, 7 male | 26.5±2.5* | 21.9±0.9* |
| High-fibre biscuit | 10 | 3 female, 7 male | 25.2±0.9* | 23.4±1.1* |
| Breakfast Rusk | 10 | 5 female, 5 male | 25.7±2.6* | 21.9±0.6* |
| Nutrient formula A | 10 | 4 female, 6 male | 20.2-38.7 | |
| Nutrient formula B | 10 | 3 female, 7 male | 21.6-28.6 | |
| Breakfast cereals (All Bran) | 6 | male | 27.8±1.5* | 22.8±0.24* |
| Breakfast cereals (Special K) | 6 | male | 27.8±1.5* | 22.8±0.24* |
| soluble fibre A | 22 | mixed | 25-45 | 20-28 |
| soluble fibre B | 12 | mixed | 26-36 | 23-27 |
| soluble fibre E | 12 | mixed | 26-36 | 23-27 |
| insoluble fibre C | 12 | mixed | 26-36 | 23-27 |
| insoluble fibre D | 12 | mixed | 26-36 | 23-27 |
| Control bread | 20 | 12 m+8 f | 27.5±17 | 24.3±1.0 |
| HM260 bread dose 1 | 20 | 12 m+8 f | 27.5±17 | 24.3±1.0 |

(continued)

| Food | Number of study subjects | Gender | Age (years) | BMI (kg/m$^2$) |
|---|---|---|---|---|
| HM260 bread dose 2 | 20 | 12 m+8 f | 27.5±17 | 24.3±1.0 |

*mean ± Standard Error of the Mean (SEM)

*In vitro* digestion of test foods

Each food was digested *in vitro* exactly according to the method as described in Example 1, with one minor adaptation. After all samples had been collected from the dialysates, 0.05% w/v sodium azide was added to the samples and reference solutions just before the overnight incubation with the brush border enzymes. The sodium azide was added to prevent any possible microbial growth.

Prediction of in *vivo* glycaemic responses

The glucose and fructose concentrations of each sample were exactly measured as described in Example 1.

As for the *in silico* model, the baseline plasma glucose and insulin values were adapted to the respective values as provided by the manufacturers of the tested foods. The adapted baseline plasma glucose concentrations ranged from 3.97 mmol/l to 5.19 mmol/l. The adapted baseline insulin concentrations ranged from 29.6 pmol/l to 45.6 pmol/l. The *in vivo* glycaemic responses to the test foods were then predicted exactly as described in Example 1. No GI values were calculated this time.

The experiment provided 15 predicted plasma glucose response curves, one for each tested food. These predicted curves were then compared to the actual *in vivo* plasma glucose response curves. The maximum glucose concentrations ($C_{max}$) of both the predicted and actual *in vivo* plasma glucose response were compared with each other, with regard to the time of appearance and the absolute glucose value. Also the average deviation of the predicted integrated area under the curve (iAUC) from the actual *in vivo* AUC was calculated, per measured time interval.

Results and conclusions

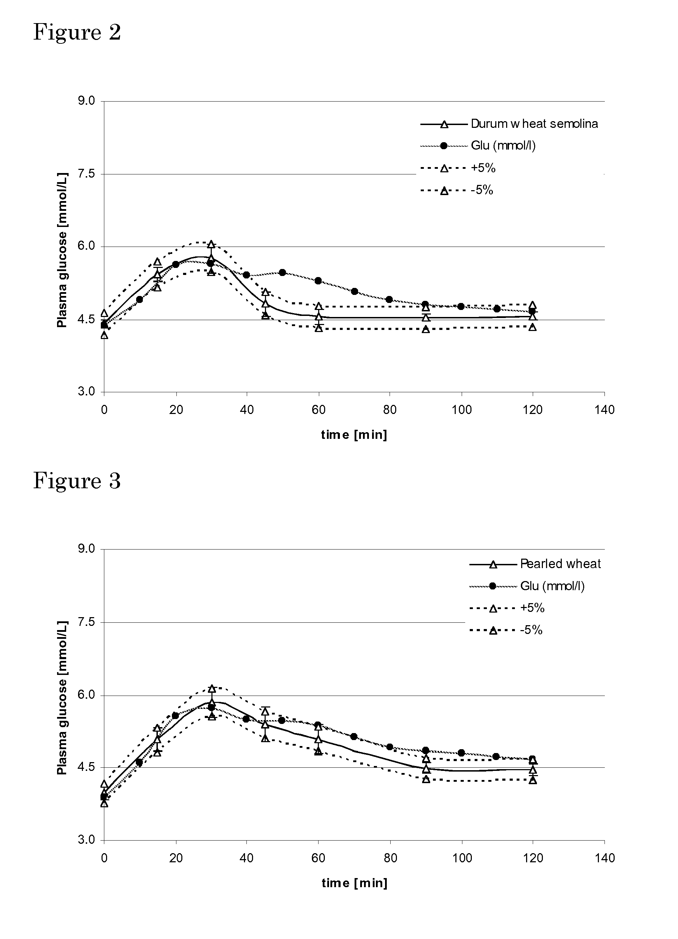

Figures 2 to 5 display some of the predicted plasma glucose response curves. In all figures, both the glucose response curve as predicted by the method according to the invention and the average actual *in vivo* glucose response curve are shown. In addition, lines are added indicating ± 5% deviation from the actual *in vivo* glucose response curve.

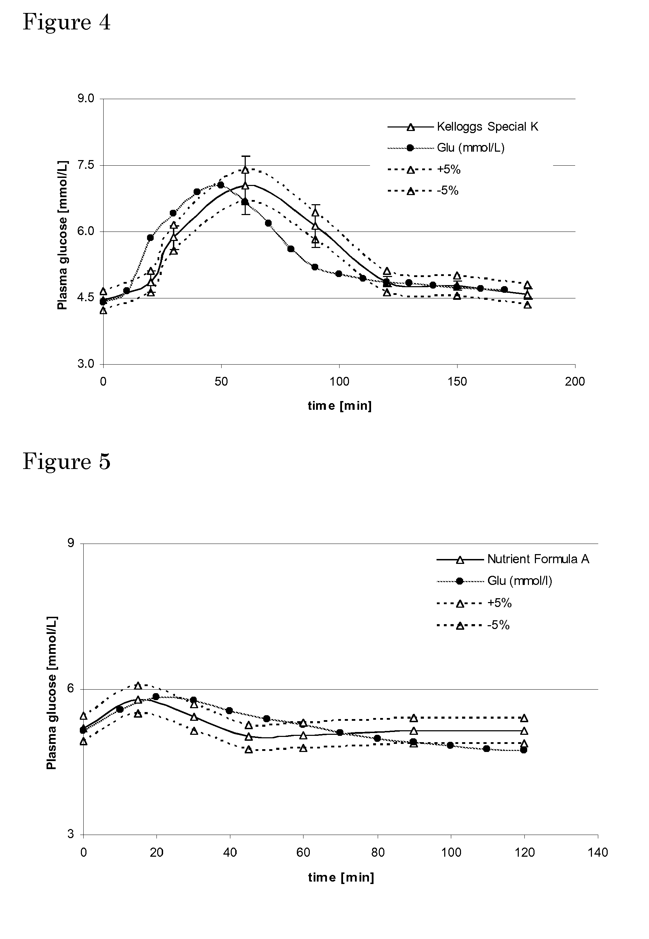

It can be observed from Figure 2 (durum wheat semolina pasta) that both the predicted and actual *in vivo* maximum glucose concentrations ($C_{max}$) occur at the same time and are of equal magnitude. A similar result can be observed in Figure 3 (parboiled pearled wheat), wherein the predicted glucose response curve closely matches and follows the actual *in vivo* glucose response curve. Figures 4 (Special K breakfast cereals) and 5 (Nutrient Formula A) show a predicted glucose response curve wherein $C_{max}$ occurs somewhat earlier (Figure 4) or later (Figure 5) than the actual *in vivo* $C_{max}$. In both figures, the predicted glucose response curve again has a similar shape as the actual *in vivo* glucose response curve.

After 50 to 90 min. the predicted glucose response curve is often slightly different from the *in vivo* glycaemic repsonse. A reason might be, that the first glucose response curve (up to $C_{max}$) is mainly driven by the rate of digestion and absorption, while at later time points the blood glucose concentrations will be mainly determined by the rate at which glucose is removed via insulin-dependent and non-insulin dependent mechanisms. (Kendall *et al.,* 2008, Effect of novel maize-based dietary fibres on postprandial glycaemia and insulinemia).

Table 6 shows the results of the comparisons of the predicted $C_{max}$ glucose values and iAUC with the actual *in vivo* $C_{max}$ and AUC. It can be observed that the predicted $C_{max}$ glucose values closely resemble the actual *in vivo* $C_{max}$ values. Larger differences can be observed when comparing the predicted iAUC versus the actual AUC. The average deviation of the predicted iAUC from the actual *in vivo* AUC, per measured time interval, ranged between 3.6% (parboiled pearled wheat) and 14.4% (soft bread).

Table 6. Comparisons of predicted $C_{max}$ and integrated area under the curve (iAUC, 0-120 minutes) with actual *in vivo* $C_{max}$ and AUC. Also shown are the average deviations of the predicted iAUC from the actual *in vivo* AUC per measured time interval. "-" means data not available.

| Test food | Predicted Cmax (mmol/l) | Actual Cmax (mmol/l) | Predicted iAUC | Actual AUC | Average deviation from actual AUC (%) |
|---|---|---|---|---|---|
| Durum wheat semolina pasta (Spaghetti Integrali) | 5.64 | 5.75 | 81.9 | 55.2 $\pm$ 6.7 | 6.0 |
| Parboiled pearled wheat | 5.74 | 5.85 | 133.3 | 114.5 $\pm$ 19.3 | 3.6 |
| Legume pasta (Spaghetti Plus) | 5.71 | 5.53 | 103.9 | 55.8 $\pm$ 12.6 | 8.4 |
| Soft bread | 7.09 | 6.31 | 185.9 | 92.1 $\pm$ 9.2 | 14.4 |
| High-fibre biscuit | 5.67 | 5.65 | 95.77 | 59.7 $\pm$ 7.7 | 6.3 |
| Breakfast Rusk | 6.25 | 6.43 | 132.9 | 95.5 $\pm$ 19.5 | 6.9 |
| Nutrient formula A | 5.83 | 5.79 | 53.5 | - | 5.0 |
| Nutrient formula B | 7.08 | 7.05 | 99.7 | - | 6.0 |
| Breakfast cereals (All Bran) | 4.96 | 5.65 | 57.3 | - | 4.8 |
| Breakfast cereals (Special K) | 7.05 | 7.04 | 180.63 | - | 7.4 |
| Soluble fibre A | 4.9 | 5.56 | 29.74 | - | 7.0 |
| Soluble fibre B | 4.78 | 5.16 | 44.3 | - | 5.3 |
| Soluble fibre E | 5.15 | 5.92 | 33.4 | - | 7.6 |
| Insoluble fibre C | 4.79 | 4.53 | 41.6 | - | 6.3 |
| Insoluble fibre D | 4.78 | 4.72 | 25.6 | - | 5.3 |
| Control bread | 6.25 | 6.24 | 125 | 93.6 $\pm$ 12.5 | 9.1 |
| HM260 bread dose 1 | 5.9 | 6.16 | 91.3 | 65.1 $\pm$ 7.8 | 10.7 |
| HM260 bread dose 2 | 5.78 | 6.06 | 86.5 | 61.5 $\pm$ 8.2 | 11.0 |

$C_{max}$ of durum wheat semolina pasta, parboiled wheat, legume pasta, high fibre biscuit, breakfast rusk, Kellogg's All Bran and Insoluble fibre D appeared during the same time interval. $C_{max}$ of the soft bread, the drink formula A, the Soluble fibre A, B, the insoluble fibre E and C occurred 5 to 10 minutes earlier *in vivo* as compared to the predicted curves, whereas $C_{max}$ for the drink formula B was measured 10 minutes later *in vivo* as compared to the predicted value.

It was mentioned above that the predicted plasma glucose curve is shifted about ten minutes to compensate for the onset of the glucose absorption *in vivo* and the experiments herein conducted. The predicted plasma glucose curve of Kellogg's Special K demonstrates this effect (Figure 4). In this experiment, the glucose measurement must have been started already with the ingestion of the test product, rather than directly thereafter, since the *in vivo* plasma glucose curves increases only after 20 minutes.

The $C_{max}$ values deviated less than 5% for durum wheat semolina pasta, parboiled pearled wheat, legume pasta, high fibre biscuit, breakfast rusk, Nutrient formula A and B, breakfast cereals (Special K), Soluble fibre B and Insoluble fibre D between the *in vivo* values and the predicted values. $C_{max}$ deviated between 5 and 15% for the soft bread, Kellogg's All Bran, Soluble fibre A, - B, - E and - C. $C_{max}$ of Soluble fibre E deviated the most with 15%, whereas the other products diverged less than 11%.

The deviation per measured time interval of both curves from each other showed a deviation not more than 9%, except the soft bread; where both curves diverged approximately 14% form each other.

For three types of bread, whether or not enriched with Hi Maize 260, the order of predicted plasma glucose curves is exactly equal to what was found *in vivo,* with the control bread resulting in the highest plasma glucose response curves and thereafter HM 260 dose 1 and subsequently HM 260 dose 2. Even the crossing between both curves of HM 260 dose 1 and HM 260 dose 2 which occurs *in vivo* after approximately 35 minutes is visible in the predicted curves at approximately t = 25 (data not shown). This indicates that the *in vitro* method according to the invention is able to detect minor differences in true digestibility and to translate this into the appropriate order of predicted plasma glucose response curves.

The first half of the predicted versus the *in vivo* plasma response curves are similar to each other until $C_{max}$ is reached. Thereafter, the predicted plasma glucose curves strive towards their steady state level, which was set at 4.63 mmol/L. In contrast, the *in vivo* glucose response plasma curves decrease below the initial fasting glucose plasma level. This is most probably due to the insulin secretion, which induces a more sudden decrease and a steeper slope of the plasma glucose curves in vivo even below the fasting plasma glucose level.

Some differences between the predicted curves and the plasma glucose levels obtained from the *in vivo* studies appear rather large. However, *in vivo* a broad inter individual range as well as very varying fasting plasma glucose baseline values (3.97 mmol/L - 5.19 mmol/L) were observed (data not shown). This depends on e.g. the age of the study population, their hunger status and their BMI. The major part of the study populations to which the predicted plasma glucose curves were compared to were young, lean and healthy adults. The *in silico* model was calibrated to predict plasma glucose levels of this type of population and the method according to the invention yields values that correspond to the average individual.

In conclusion, the tested plasma glucose curves were reliably predicted *in vitro* with a deviation of not more than 9%. The often very high inter subject variability that occurs *in vivo* is not observed in the methods according to the invention, as the conditions are highly standardized and reproducible. Therefore the methods according to the invention provide very useful tools to study the glucose response *in vitro,* and to adequately predict the *in vivo* glycaemic response to a food.

**Claims**

1. A method for predicting the *in vivo* glycaemic response to carbohydrates in a food wherein the food is digested *in vitro,* **characterized in that** the food is digested in more than one compartment.

2. Method according to claim 1, wherein said food is digested in two compartments.

3. Method according to claim 1 or 2, comprising digesting said food with at least one enzyme selected from the group of amylases, lipases, proteinases, including but not limited to pepsin, trypsin, chymotrypsin, pancreatin and brush border enzymes.

4. Method according to any one of claims 1 to 3, comprising digesting said food at a specific pH value that may be different between compartments.

5. Method according to any one of claims 1 to 4, comprising applying pressure to said food.

6. Method according to claim 5, wherein said pressure is applied in a peristaltic manner.

7. Method according to claim 5 or 6, wherein said pressure is mechanically induced.

8. Method according to any one of claims 1 to 7, comprising transferring said food from one compartment to another in a peristaltic motion.

9. Method according to claim 8, wherein said peristaltic motion is mechanically induced.

10. Method according to any one of claims 1 to 9, comprising collecting at least one sample from said food during and/or after digestion.

**11.** Method according to claim 10, comprising further *in vitro* digesting at least one carbohydrate in said at least one sample collected from said food.

**12.** Method according to claim 10 or 11, comprising measuring the amount of at least one monosaccharide, preferably selected from the group consisting of glucose, fructose and galactose in said at least one sample collected from said food.

**13.** Method according to claim 12, comprising calculating the *in vitro* glucose response to said food.

**14.** Use of a method according to any one of claims 1 to 13 for determining the suitability of a food for a subject who would benefit from a normalization of blood glucose levels.

**15.** Use according to claim 14, wherein said subject is suffering from or having increased risk for developing at least one syndrome selected from the group of glucose intolerance, insulin resistance, diabetes mellitus, syndrome X (or metabolic syndrome), cardiovascular disease, overweight and obesity.

# Figure 1

**Plasma Glucose**

**plasma Insulin**

## Figure 2

## Figure 3

# Figure 4

# Figure 5

EUROPEAN SEARCH REPORT

Application Number

EP 09 16 2517

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | ENGLYST KLAUS N; ET AL: "Rapidly available glucose in foods: An in vitro measurement that reflects the glycemic response" AMERICAN JOURNAL OF CLINICAL NUTRITION, AMERICAN SOCIETY FOR CLINICAL NUTRITION, BETHESDA, MD, US, vol. 69, no. 3, 1 March 1999 (1999-03-01), pages 448-454, XP002353205 ISSN: 0002-9165 * the whole document * ----- | 1-15 | INV. G01N33/66 |
| A | BROUNS F; ET AL: "Glycaemic index methodology" NUTRITION RESEARCH REVIEWS, CAMBRIDGE UNIVERSITY PRESS, CAMBRIDGE, GB, vol. 18, 1 January 2005 (2005-01-01), pages 145-171, XP009121970 ISSN: 0954-4224 * the whole document * ----- | 1-15 | |
| A,D | WO 2009/006155 A1 (KRAFT FOODS GLOBAL BRANDS LLC) 8 January 2009 (2009-01-08) * claims 1-20; example 1 * ----- | 1-15 | TECHNICAL FIELDS SEARCHED (IPC) G01N |

The present search report has been drawn up for all claims

| Place of search | Date of completion of the search | Examiner |
|---|---|---|
| Munich | 7 September 2009 | Moreno de Vega, C |

## ANNEX TO THE EUROPEAN SEARCH REPORT
## ON EUROPEAN PATENT APPLICATION NO.

EP 09 16 2517

This annex lists the patent family members relating to the patent documents cited in the above-mentioned European search report.
The members are as contained in the European Patent Office EDP file on
The European Patent Office is in no way liable for these particulars which are merely given for the purpose of information.

07-09-2009

| Patent document cited in search report | Publication date | Patent family member(s) | Publication date |
|---|---|---|---|
| WO 2009006155 A1 | 08-01-2009 | NONE | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009006155 A
- US 5525305 A
- EP 0642382 B1

### Non-patent literature cited in the description

- **Jenkins et al.** *American Journal of Clinical Nutrition,* 1981, vol. 34, 362-366
- **Brouns et al.** *Nutrition Research Reviews,* 2005, vol. 18, 145-171
- **Temelkova-Kurktschiev et al.** *Diabetes Care,* 2000, vol. 23 (12), 1830-1834
- **Balkau et al.** *Diabetes Care,* 1998, vol. 21 (3), 360-367
- **Englyst et al.** *British Journal of Nutrition,* 1996, vol. 75, 327-337
- **Englyst et al.** *American Journal of Clinical Nutrition,* 1999, vol. 69, 448-454
- **Garsetti et al.** *J Am Coll Nutr,* 2005, vol. 24, 441-447
- **Brand-Miller et al.** *European Journal of Clinical Nutrition,* 2004, vol. 58, 700-701
- **Wallace et al.** *Diabetes Care,* 2004, vol. 27 (6), 1487-1495
- **Juntunen et al.** *Am. J. Clin. Nutr.,* 2002, vol. 75, 254-262
- **Lee et al.** *Eur J Clin Nutr,* 1998, vol. 52, 924-928
- **Hofman et al.** *Eur J Clin Nutr,* 2004, vol. 58, 1553-1556
- **Hofman.** *Asia Pac J Clin Nutr,* 2006, vol. 15 (3), 412-417
- **Juntunen et al.** *Am J Clin Nutr,* 2002, vol. 75, 254-262
- **Foster-Powell et al.** *Am J CLin Nutr,* 2002, vol. 76 (1), 5-56
- **Liljeberg et al.** *Eur J Clin Nutr,* 1992, vol. 46 (8), 561-575
- **Genuth et al.** *Diabetes Care,* 2003, vol. 26, 3160-3167